(19) Europäisches Patentamt

European Patent Office

Office européen des brevets

(11) Publication number: **0 222 661 B1**

## EUROPEAN PATENT SPECIFICATION

(45) Date of publication of patent specification: **08.07.92**    (51) Int. Cl.[5]: **C12Q 1/04**

(21) Application number: **86402431.0**

(22) Date of filing: **30.10.86**

(54) **Medium composition for testing the citrate-utilizing capability of microorganisms.**

(30) Priority: **31.10.85 JP 242972/85**

(43) Date of publication of application:
**20.05.87 Bulletin 87/21**

(45) Publication of the grant of the patent:
**08.07.92 Bulletin 92/28**

(84) Designated Contracting States:
**BE DE FR GB IT NL SE**

(56) References cited:
**EP-A- 0 161 481**
**LU-A- 78 801**
**US-A- 3 830 703**

**DIFCO MANUAL OF DEHYDRATED CULTURE MEDIA AND REAGENTS FOR MICROBIOLOGICAL AND CLINICAL LABORATORY PROCEDURES, 9th edition, 1977, page 182, DIFCO Laboratories, Detroit, Michigan, US**

(73) Proprietor: **TERUMO KABUSHIKI KAISHA trading as TERUMO CORPORATION**
**44-1, 2-chome, Hatagaya Shibuya-Ku**
**Tokyo 151(JP)**

(72) Inventor: **Shigematsu, Takashi**
**2-3-4-202, Nagayama**
**Tama-shi Tokyo(JP)**

(74) Representative: **Descourtieux, Philippe et al**
**CABINET BEAU de LOMENIE 55 rue d'Amsterdam**
**F-75008 Paris(FR)**

Rank Xerox (UK) Business Services

**Description**

Background of the Invention

1. Field of the Invention

This invention relates to a medium composition for testing the capability of microorganisms to utilize citrates. More particularly, it is concerned with a medium composition for testing the citrate-utilizing capability of glucose-nonfermentative Gram-negative bacilli.

In order to apply an appropriate therapy for microbial infections it is important to identify pathogenic bacteria, conduct sensitivity tests and determine a drug active against the pathogenic bacteria. In the identification of pathogenic bacteria for the above purpose, a variety of examinations of biochemical properties is carried out, among which is the test of the citrate-utilizing capability of glucose-nonfermentative Gram-negative bacilli.

In the test, the identification of said bacteria is carried out by utilizing their citrate-decomposing capability. The medium composition of the invention can preferably be used for such a test.

2. Description of Prior Art

A medium for the above-mentioned test heretofore known is Simons citrate agar medium having the composition shown below:

| | |
|---|---|
| $NH_4 H_2 PO_4$ | 1.0 (g) |
| $K_2 HPO_4$ | 1.0 |
| NaCl | 5.0 |
| Trisodium citrate | 2.0 |
| $MgSO_4 \cdot 7H_2 O$ | 0.2 |
| Agar | 15.0 |
| Bromthymol Blue | 0.08 |
| Distilled water | 1000 (ml) |

Such a medium is disclosed, for example, in the US Patent n° 3 830 703.

Since the above medium is an agar medium, the reaction rate is slow and judgment requires 1-3 days.

In order to treat microbial infections at earlier stages it is necessary to carry out the identification of microorganisms as soon as possible, and development of the medium which enables identification of microorganisms in a shorter period of time has been desired. In some cases, however, the judgment is made inevitably on the next day for the convenience of operation. In such a case, it is desirable to employ a medium in which strict fixation of cultivation period of time is not required, and extension of the cultivation period of time by certain reasons does not cause an excess reaction.

Summary of the Invention

It is an object of the present invention to provide a medium composition for testing the citrate utilization capability of glucose-nonfermentative Gram-negative bacilli which enables the identification by cultivation for a shorter period of time as well as makes it possible to identify the microorganism correctly after cultivation for a longer period of time.

A further object of the invention is to provide a medium composition for the above described test in which the color reaction is clear, and the judgment is easy.

According to the invention, there is, provided a medium composition comprising, in parts by weight, 1.0 part of a tri-alkali metal citrate, 0.1-0.5 (preferably 0.125-0.45) part of di-alkali metal hydrogenphosphate, 0.1-0.5 (preferably 0.125-0.45) part of ammonium dihydrogenphosphate, 0.04-0.3 (preferably 0.05-0.2) part of magnesium salt of a mineral acid, 0.01-0.1 (preferably 0.02-0.08) part of a pH indicator, and being devoid of agar.

Detailed Description of the Invention

The tri-alkali metal citrate in the medium composition of the invention is the substrate, and pH value of the medium will be increased due to formation of basic substances if the salt is decomposed by the test organism. Citrate utilization capability of the test organism is judged by detecting the change by means of the pH indicator. The tri-alkali metal citrate is employed usually in the form of a hydrate. The preferred tri-alkali metal citrate is trisodium citrate dihydrate.

The di-alkali metal hydrogenphosphate and the ammonium dihydrogenphosphate are pH-adjusting agents, which are mixed at a new ratio as well as at a level lower than that in the prior art compositions. As example of di-alkali metal hydrogenophosphate it may be cited the di-sodium or di-potassium hydrogenophosphate. Therefore, pH change is produced more easily, and the color reaction by a pH indicator occurs more easily and rapidly. The pH is also fixed within such a range as being optimal for the growth of microorganism. In the magnesium salt of the mineral acid, hydrochloric, sulfuric, nitric or the like acid is preferably employed. There is no particular limitation to the nature of the pH indicator, for which Phenol Red, Bromthymol Blue, Bromcresol Purple, Cresol Red, Bromphenol Red and the like are suitable. Especially, use of Bromthymol Blue produces clear coloring so that judgment for positive or negative reaction is easily made with a smaller amount of the test sample. In addition, it is desirable to use an alkali metal salt as an osmotic pressure-controlling agent, for which sodium chloride, potassium chloride and the like are preferable.

Proportions of the components in the medium of the invention described above are critical, and particularly the pH-adjusting agents are fixed in such a way that said composition has a pH between 6.5 and 7.0, and preferably between 6.7 and 6.8, and pH of the medium will be 7.2 or higher when the citrate substrate is decomposed.

The amount of the pH indicator used is somewhat variable depending upon the nature thereof. For example, Phenol Red is preferably employed in an amount from 0.01 to 0.1 part.

The culture medium composition of the invention is employed by dissolving the components in amounts as defined above in water. When simultaneous examinations for a number of tests using a multiwell plate are to be carried out, as are usual in the biochemical test, a solution of the composition according to the invention is poured into a well in a test plate and dried to prepare a dry medium. In carrying out the test, an aqueous suspension of the microorganism to be tested in a predetermined amount is poured into each of the well, and cultivation is carried out at 30°C for a predetermined period of time. The culture medium is inoculated with approximately $7.5 \times 10^7$ organisms per 50 $\mu$l of the medium as calculated as a liquid medium if the judgment is desired to be made by 4- to 5-hour cultivation, and preferably with approximately 1.5 to $10^7$ organisms if the judgment is desired to be made after 18- to 22-hour cultivation. As set forth above, the judgment can be either in a short period of time or on the day subsequent to the inoculation.

Examples are given below of the test of citrate utilization capability of microorganisms using the medium composition of the invention.

Examples

A medium composition of the invention with a composition as described in Table 1 was dissolved in 1 lit. of water to prepare the media 1-3.

Table 1

| Component | Medium 1 | Medium 2 | Medium 3 |
|---|---|---|---|
| $K_2HPO_4$ | 0.3 | 0.5 | 0.7 |
| $NH_4H_2PO_4$ | 0.3 | 0.5 | 0.7 |
| $MgSO_4 \cdot 7H_2O$ | 0.2 | 0.2 | 0.2 |
| NaCl | 5 | 5 | 5 |
| Trisodium citrate dihydrate | 1 | 1.5 | 2 |
| Bromthymol Blue | 0.08 | 0.08 | 0.08 |
| Distilled water | 1000 | 1000 | 1000 |

(g)

The media 1-3 described above were poured respectively in an amount of 50 $\mu$l into a well of a multiwell plate. The media were dried at 40°C. A culture of various microorganisms on an agar plate, obtained by cultivation at 30°C for 18-24 hours, was suspended in sterilized distilled water to approximately 1.5 x $10^9$ microorganisms per ml for the 4-hour run and 3 x $10^8$ microorganisms per ml for the 20-hour run. Each well was then inoculated with 50 $\mu$l of the suspension, and the plate was incubated at 30°C for a predetermined period of time. A cultivation was carried out by the prior art method using Simons medium. Positive or negative reaction for the citrate utilization capability was judged by change in color of the medium. Results are shown in Table 2.

Table 2

| Medium / Cultivation time (hr) Microorganism | Medium 1 | | Medium 2 | | Medium 3 | | Control medium | |
|---|---|---|---|---|---|---|---|---|
| | 4 | 20 | 4 | 20 | 4 | 20 | 4 | 20 |
| Pseudomonas putida ATCC 12633 | + | + | + | + | + | + | − | + |
| Pseudomonas aeruginosa ATCC 10145 | + | + | + | + | + | + | − | + |
| Pseudomonas cepacia ATCC 27515 | + | + | + | + | + | + | − | + |
| Flavobacterium meningosepticum ATCC 15253 | − | − | − | − | − | − | − | − |
| Xanthomonas maltophilia ATCC 12637 | − | − | − | − | − | − | − | − |

As apparent from Table 2, use of the medium of the present invention in the test for citrate-utilizing capability of glucose-nonfermentative Gram-negative bacilli enables correct identification of the microorganism independent of the cultivation time. Therefore, the identification may be made either on the same day or on the next day for the convenience of examination operation. On the contrary, use of the control medium needs cultivation for at shortest 24 hours thereby making the identification on the same day infeasible. With the control medium, for example, 4-hour cultivation of Pseudomonas putida ATCC 12633, Pseudomonas aeruginosa ATCC 10145 and Pseudomonas cepacia ATCC 27515 gives negative reaction, and after the 24-hour cultivation the reaction becomes positive. On the contrary, use of the medium of the invention enables correct judgment by the 4-hour cultivation with any of the microorganisms.

## Claims

1. A medium composition for testing the citrate-utilizing capability of glucose-non fermentative Gram-negative bacilli comprising, in parts by weight, 1.0 part of tri-alkali metal citrate, 0.1 - 0.5 part of di-alkali metal hydrogenphosphate, 0.1 - 0.5 part of ammonium dihydrogenphosphate, 0.04 - 0.3 part of magnesium salt of a mineral acid, 0.01 - 0.1 part of a pH indicator and being devoid of agar.

2. A medium composition according to Claim 1 which comprises, in parts by weight, 1.0 part of tri-alkali metal citrate, 0.125 - 0.45 part of di-alkali metal hydrogenphosphate, 0.125 - 0.45 part of ammonium dihydrogenphosphate, 0.05 - 0.2 part of magnesium salt of a mineral acid and 0.02 - 0.08 part of a pH indicator.

3. A medium composition according to Claim 1 or 2 wherein the tri-alkali metal citrate is trisodium citrate.

4. A medium composition according to Claims 1 to 3 wherein the di-alkali metal hydrogenphosphate is disodium or dipotassium hydrogenphosphate.

## Revendications

1. Composition de milieu pour tester la capacité d'un bacille à Gram-négatif ne fermentant pas le glucose à utiliser les citrates comprenant, en parties en poids, 1,0 partie de citrate de tri-métal alcalin, 0,1-0,5 partie de phosphate de di-métal alcalin, 0,1-0,5 partie de phosphate de monoammonium, 0,04-0,3 partie d'un sel de magnésium d'un acide minéral, 0,01-0,1 partie d'un indicateur de pH et qui est exempte d'agar.

2. Une composition de milieu selon la revendication 1 qui comprend, en parties en poids, 1,0 partie de citrate de trimétal alcalin, 0,125-0,45 partie de phosphate de di-métal alcalin, 0,125-0,45 partie de phosphate de monoammonium, 0,05-0,2 partie de sel de magnésium d'un acide minéral et 0,02-0,8 partie d'un indicateur de pH.

3. Une composition de milieu selon la revendication 1 ou 2 dans laquelle le citrate de tri-métal alcalin est le citrate trisodique.

4. Une composition de milieu selon les revendications 1 à 3 dans laquelle le phosphate de di-métal alcalin est le phosphate disodique ou dipotassique.

## Patentansprüche

1. Mediumzusammensetzung zur Bestimmung der Citratverwendungsfähigkeit von Glucose nicht abbauenden gram-negativen Bazillen, die in Gewichtsteilen enthält: 1,0 Teil Trialkalimetallcitrat, 0,1 - 0,5 Teile Dialkalimetallhydrogenphosphat, 0,1 - 0,5 Teile Ammoniumdihydrogenphosphat, 0,04 - 0,3 Teile eines Magnesiumsalzes einer anorganischen Säure, 0,01 - 0,1 Teile eines pH-Indikators, und agarfrei ist.

2. Mediumzusammensetzung nach Anspruch 1, die in Gewichtsteilen enthält: 1,0 Teil Trialkalimetallcitrat, 0,125 - 0,45 Teile Dialkalimetallhydrogenphosphat, 0,125 - 0,45 Teile Ammoniumdihydrogenphosphat, 0,05 - 0,2 Teile eines Magnesiumsalzes einer anorganischen Säure und 0,02 - 0,08 Teile eines pH-Indikators.

3. Mediumzusammensetzung nach Anspruch 1 oder 2, worin das Trialkalimetallcitrat Trinatriumcitrat ist.

4. Mediumzusammensetzung nach den Ansprüchen 1 bis 3, worin das Dialkalimetallhydrogenphosphat Dinatrium- oder Dikaliumhydrogenphosphat ist.